# EUROPÄISCHE PATENTSCHRIFT

(11) **EP 1 649 816 B1**
(45) Veröffentlichungstag und Bekanntmachung des Hinweises auf die Patenterteilung: **18.07.2007**
(21) Anmeldenummer: 05022552.3
(22) Anmeldetag: 15.10.2005
(51) Int. Cl.: A61B 17/02

(54) **Auslenkbares endoskopisches Instrument**
Deflectable endoscopic instrument
Instrument endoscopique déviable

(30) Priorität: 19.10.2004 DE 102004052204
(43) Veröffentlichungstag der Anmeldung: 26.04.2006
(73) Patentinhaber: Karl Storz GmbH & Co. KG, 78532 Tuttlingen (DE)
(72) Erfinder: Bacher, Uwe, 78532 Tuttlingen (DE)
(74) Vertreter: Weller, Wolfgang

(56) Entgegenhaltungen:
- WO-A-03/094744
- DE-A1- 19 840 880

## Beschreibung

Die Erfindung betrifft ein endoskopisches Instrument mit einem Schaft, der ein proximales und ein distales Ende aufweist, wobei am proximalen Ende des Schafts ein Griff mit einer Handhabe und am distalen Ende des Schafts ein Werkzeug angeordnet ist, das zumindest eine geöffnete und eine geschlossene Stellung aufweist, wobei der Schaft zumindest zwei Bereiche aufweist, einen ersten Bereich und einen zweiten gegenüber dem ersten auslenkbaren Bereich, wobei der zweite auslenkbare Bereich das Werkzeug aufweist, und wobei das Instrument ferner ein Betätigungselement aufweist, über das die Handhabe mit dem zweiten auslenkbaren Bereich in Wirkverbindung steht und mit dem eine Auslenkung des zweiten auslenkbaren Bereichs auslösbar ist.

Ein derartiges endoskopisches Instrument ist zum Beispiel aus der EP 0 582 295 B1 bekannt.

Solche endoskopischen Instrumente werden bei der minimalinvasiven Chirurgie eingesetzt. Hierbei werden unter Sichtkontrolle durch ein Endoskop über kleine Einschnitte chirurgische Instrumente in den Körper eines Patienten eingebracht, um dort Manipulationen vorzunehmen. Dadurch, dass die Operationsstelle bei der minimalinvasiven Chirurgie nicht mehr vollständig freigelegt werden muss, sind deutlich kleinere Einschnitte und damit eine deutlich geringere Belastung des Patienten notwendig. Dies verkürzt die Zeit, die sich ein Patient stationär im Krankenhaus aufhalten muss und macht einen Krankenhausaufenthalt teilweise sogar überflüssig. Außerdem ist die Möglichkeit, dass Krankheitserreger in den Körper des Patienten eindringen, geringer. Somit können heutzutage mit Hilfe minimalinvasiver Chirurgie Eingriffe ambulant durchgeführt werden, für die früher ein teilweise langwieriger Krankenhausaufenthalt notwendig war.

Um ein Einführen durch einen kleinen Einschnitt möglich zu machen, weisen solche endoskopischen Instrumente im Allgemeinen eine langgestreckte Form mit einem möglichst kleinen Querschnitt auf.

Es hat sich jedoch gezeigt, dass solche Instrumente in ihren Einsatzmöglichkeiten beschränkt sind, da ein langgestrecktes Instrument nur einen begrenzten Bereich um sein distales Ende herum erreichen kann. Bereiche, die sich zum Beispiel in einem 90°-Winkel gegenüber dem Instrument befinden, können nicht erreicht werden. Auch ist es mit einem solchen Instrument nicht möglich, um ein Hindernis herumzureichen.

Somit ist es wünschenswert, wenn ein solches endoskopisches Instrument einen Bereich aufweist, der gegenüber dem Endoskop auslenkbar ist. Somit können zum Beispiel Stellen erreicht werden, die hinter einem Organ liegen, ohne dass dadurch der Querschnitt des Endoskops bei der Einführung vergrößert wird.

Weiterhin weisen die endoskopischen Instrumente an ihrem distalen Ende Werkzeuge auf, mit denen Manipulationen im Körper des Patienten durchgeführt werden können. Diese Instrumente weisen häufig eine geöffnete und eine geschlossene Stellung auf. Die geschlossene Stellung kann zum Beispiel zur Minimierung des Querschnitts des Instruments beim Einführen in den Körper des Patienten dienen oder dazu dienen, beim Einführen in den Körper des Patienten an den Werkzeugen befindliche Klingen zu maskieren, um Verletzungen zu vermeiden.

Solche endoskopischen Instrumente können zum Beispiel als sogenannte Retraktoren verwendet werden. Sie dienen hierbei dazu, ein Organ, zum Beispiel die Leber, im Körper des Patienten zu bewegen, so dass mit Hilfe eines Endoskops die Rückseite des Organs untersucht werden kann.

Bei einem solchen Retraktor ist sowohl ein Auslenken eines Bereichs des Schaftes als auch das Öffnen und Schließen des Werkzeugs wichtig. Das Auslenken eines Bereichs des Schaftes dient dazu, das Werkzeug hinter dem Organ zu platzieren, so dass dieses bewegt werden kann.

Das Öffnen und Schließen des Werkzeugs dient dazu, in geschlossenen Zustand beim Einführen einen möglichst geringen Querschnitt zu bilden, während es in geöffneter Stellung als Retraktor dazu dient, eine möglichst große Auflagefläche für das Organ zu bieten, um die angewendete Kraft auf einen möglichst großen Bereich zu verteilen. Hierdurch werden Verletzungen am Organ durch das Instrument minimiert.

Die Schnittstelle zwischen dem ersten und dem zweiten auslenkbaren Bereich kann jede Form annehmen, die eine zumindest in eine Richtung auslenkbare Verbindung schafft. Beispiele für solche Verbindungen sind Schwenkzapfen, Schraubenfedern, Scharniere oder Kombinationen davon.

Der Griff und die Handhabe können in jeder aus dem Stand der Technik bekannten Form ausgeführt sein. Der Griff kann als Pistolengriff, Scherengriff, als stabförmiger Griff oder in jeder Form ausgeführt sein, die ein Manipulieren des Instruments erlaubt. Die Handhabe kann zum Beispiel in Form eines Scherengriffs, eines Zug- oder Schubelements oder als Drehknopf ausgebildet sein, wobei bei der Auslegung der Handhabe die Form des Griffs berücksichtigt werden sollte.

Solche endoskopischen Instrumente sind zum Beispiel aus der EP 582 295 B1 bekannt. In diesem Dokument sind verschiedene endoskopische Instrumente dargestellt, die einen langgestreckten Schaft mit einem ersten Bereich und einem zweiten gegenüber dem ersten auslenkbaren Bereich aufweisen.

Am distalen Ende des Schafts sind ferner Werkzeuge mit einer geöffneten und einer geschlossenen Stellung angeordnet. Diese Werkzeuge sind an dem zweiten auslenkbaren Bereich angeordnet.

Es besteht ferner eine Wirkverbindung zwischen dem auslenkbaren Bereich und einem am Griff angeordneten Stellorgan.

In den in diesem Dokument dargestellten Fällen ist jedoch nicht der zweite auslenkbare Bereich mit der Handhabe verbunden, sondern das Werkzeug. Der zweite auslenkbare Bereich ist mit einem zweiten Betätigungselement verbunden, das heißt die Auslenkung des auslenkbaren Bereichs und das Öffnen und Schließen des Werkzeugs erfolgen durch zwei unterschiedliche Antriebe.

Aus dieser Anordnung ergibt sich, dass diese endoskopischen Instrumente nicht im Einhandbetrieb verwendbar sind. Dies ist allerdings häufig wünschenswert, so dass ein Operateur die zweite Hand zum Beispiel zum Nachführen eines Endoskop zur Sichtkontrolle frei hat.

Es hat sich ferner gezeigt, dass es häufig schwierig ist, das Auslenken des auslenkbaren Bereichs und das Öffnen und Schließen der Werkzeuge mit zwei Händen aufeinander abzustimmen.

Die aus diesem Dokument bekannten Instrumente sind außerdem auf Grund der zwei vorhandenen Antriebe mechanisch komplex, wobei die große Anzahl an benötigten Bauteilen aus den in diesem Dokument dargestellten Explosionszeichnungen hervorgeht.

Eine solche mechanische Komplexität macht diese Instrumente in der Herstellung kostenintensiv. Ferner ist ein Auseinanderbauen zum Beispiel zu Reinigungszwecken kompliziert und auf Grund der vielen ineinandergreifenden Einzelteile existiert eine große Anzahl von Ritzen und Nischen, in die Körperflüssigkeiten, Bakterien oder andere Verunreinigungen eindringen können.

Es wäre ein endoskopisches Instrument wünschenswert, bei dem mit einem einzigen Antrieb sowohl das Auslenken des auslenkbaren Bereichs als auch das Öffnen und Schließen des Werkzeugs zu bewerkstelligen ist. Somit wäre ein solches Instrument im Einhandbetrieb verwendbar.

Außerdem wäre damit ein gleichförmiges aufeinander abgestimmtes Auslenken des auslenkbaren Bereichs und Öffnen und Schließen des Werkzeugs möglich.

Außerdem sollte ein solches Instrument mechanisch möglichst einfach sein.

Erfindungsgemäß wird die Aufgabe dadurch gelöst, dass bei dem endoskopischen Instrument der erste Bereich oder das Betätigungselement und das Werkzeug über ein Steuerelement verbunden sind, mittels dem das Werkzeug im Laufe einer Auslenkung des zweiten auslenkbaren Bereichs zwischen der geschlossenen und der offenen Stellung hin- und herbewegbar ist, so dass durch die Bewegung des Betätigungselements sowohl die Auslenkung des zweiten auslenkbaren Bereichs als auch ein Öffnen/Schließen des Werkzeugs bewerkstelligbar ist.

Durch die oben genannte Maßnahme werden die Auslenkung des zweiten auslenkbaren Bereichs und das Öffnen und Schließen des Werkzeugs aneinander gekoppelt. Somit können beide Bewegungen durch einen einzigen Antrieb bewerkstelligt werden. Dadurch wird ein endoskopisches Instrument mit einem auslenkbaren Bereich und einem Werkzeug mit zumindest einer geöffneten und einer geschlossenen Stellung geschaffen, das im Einhandbetrieb verwendbar ist.

Die Verbindung des ersten Bereichs bzw. des Betätigungselements und des Werkzeugs über ein Steuerelement sorgt weiterhin dafür, dass die Auslenkung des zweiten auslenkbaren Bereichs und das Öffnen und Schließen des Werkzeugs als eine aufeinander abgestimmte Bewegung erfolgt.

Dadurch, dass das erfindungsgemäße endoskopische Instrument nur noch einen Antrieb aufweist, weist es außerdem deutlich weniger Bauteile auf als ein endoskopisches Instrument gemäß dem Stand der Technik. Somit ist ein erfindungsgemäßes Instrument kostengünstiger in der Herstellung und einfacher zum Beispiel zur Reinigung auseinander zu bauen.

Ferner kann, da nur ein Betätigungselement benötigt wird, der Querschnitt des Schafts des Instruments, in dem das Betätigungselement verläuft, kleiner gehalten werden.

Das Steuerelement kann hierbei sowohl als ein einzelnes Bauteil als auch als Baugruppe aus Bauteilen ausgebildet sein. Ist das Steuerelement ein einzelnes Bauteil, kann es sich zum Beispiel um eine Steuerstange oder einen Seilzug handeln. Als Baugruppe können zum Beispiel ineinandergreifende Zahnräder oder Kombinationen aus Zahnrädern und Zahnstangen verwendet werden.

In einer Ausgestaltung der Erfindung ist das Steuerelement als Steuerstange ausgebildet.

Eine Steuerstange hat den Vorteil, dass sie eine einstückige Verbindung zwischen dem ersten Bereich bzw. dem Betätigungselement und dem Werkzeug herstellt, was zu einer besonders festen Verbindung und einer effizienten Kraftübertragung zwischen den beiden Bauteilen führt. Eine Steuerstange ist weiterhin einfach und preisgünstig in der Herstellung.

In einer weiteren Ausgestaltung der Erfindung sind der erste Bereich und der zweite auslenkbare Bereich über eine Schwenkachse verbunden.

Obwohl es möglich ist, den ersten und den zweiten auslenkbaren Bereich über jedes flexible Bauteil, wie zum Beispiel eine Schraubenfeder, zu verbinden, ist eine Schwenkachse eine bevorzugte Ausführungsform, da dadurch ein fester Verbindungspunkt zwischen den beiden Bauteilen geschaffen wird. Die Auslenkung des zweiten auslenkbaren Bereichs erfolgt somit an einem genau definierten Punkt.

Dadurch, dass durch eine Schwenkachse eine feste Verbindung zwischen dem ersten und dem zweiten auslenkbaren Bereich geschaffen wird, wird außerdem die Kraftübertragung von dem Betätigungselement auf den zweiten auslenkbaren Bereich effizienter gemacht, da eine solche Verbindung keine weiteren Freiheitsgrade mehr aufweist, in deren Richtung der auslenkbare Bereich ausweichen könnte.

In einer Ausgestaltung der Erf.indung ist das Werkzeug um eine Drehachse bewegbar am zweiten auslenkbaren Bereich angeordnet.

Ähnlich wie die Anordnung des ersten und des zweiten auslenkbaren Bereichs um eine Schwenkachse ist die Anordnung des Werkzeugs um eine Drehachse eine besonders feste Verbindung zwischen dem Werkzeug und dem zweiten auslenkbaren Bereich. Dadurch wird ebenfalls die Kraftübertragung vom Steuerelement an das Werkzeug besonders effektiv gemacht, da das Werkzeug wiederum keine weiteren Möglichkeiten zum Ausweichen hat.

In einer weiteren Ausgestaltung der oben genannten Maßnahmen ist ein erstes Ende des Steuerelements an einer Stelle mit dem ersten festen Bereich verbunden, die im Abstand zur Schwenkachse liegt.

Durch eine solche exzentrische Verbindung des Steuerelements mit dem ersten festen Bereich kann eine kreisförmige Bewegung des zweiten auslenkbaren Bereichs, und damit des daran angebrachten Werkzeugs, relativ zu dem ersten Bereich einfach in eine Linearbewegung umgesetzt werden. Das Steuerelement und der zweite auslenkbare Bereich bewegen sich während der Auslenkung auf Kreisbahnen um zwei exzentrische Mittelpunkte. Hierdurch kommt es zu Veränderungen im Abstand zwischen den beiden durch das Steuerelement bzw. den zweiten auslenkbaren Bereich beschriebenen Kreisbahnen.

Da nun das Steuerelement eine feste Länge aufweist, ändert sich der Abstand des ersten Endes und damit der Abstand eines zweiten Endes des Steuerelements von einem gegebenen Punkt auf dem zweiten auslenkbaren Bereich während der Auslenkung. Diese lineare Relativbewegung zwischen dem Steuerelement und dem zweiten auslenkbaren Bereich kann dazu benutzt werden, das Werkzeug zwischen einer offenen und einer geschlossenen Stellung hin und her zu bewegen.

In einer weiteren Ausgestaltung der oben genannten Maßnahme ist ein zweites Ende des Steuerelements an einer Stelle mit dem Werkzeug verbunden, die im Abstand zur Drehachse liegt.

Durch eine exzentrische Anordnung des Steuerelements gegenüber der Schwenkachse, die den ersten mit dem zweiten auslenkbaren Bereich verbindet, wird, wie zuvor beschrieben, zwischen jedem Punkt des zweiten auslenkbaren Bereichs und dem Steuerelement eine lineare Relativbewegung hervorgerufen. Somit wird auch zwischen der Drehachse, mit der das Werkzeug an dem zweiten auslenkbaren Bereich angeordnet ist, und dem Steuerelement eine lineare Relativbewegung hervorgerufen.

Im Falle einer Verbindung des Betätigungselements mit dem ersten Ende des Steuerelements führt das Steuerelement neben seiner kreisförmigen Bewegung eine Linearbewegung aus, da sich sein Befestigungspunkt an dem sich linear bewegenden Betätigungselement befindet. Somit führt der Mittelpunkt der Kreisbewegung des Steuerelements relativ zur Schwenkachse und damit zu jedem Punkt auf dem zweiten auslenkbaren Bereich eine lineare Relativbewegung aus. Die Kombination aus Linearbewegung des Befestigungspunkts und der Kreisbewegung des Steuerelements führt zu einer linearen Relativbewegung des zweiten Endes des Steuerelements gegenüber dem zweiten auslenkbaren Bereich und somit auch zu der fest mit dem zweiten auslenkbaren Bereich verbundenen Drehachse.

Wird nun das zweite Ende des Steuerelements an einer Stelle mit dem Werkzeug verbunden, die im Abstand zur Drehachse liegt, führt eine Linearbewegung des Steuerelements relativ zum zweiten auslenkbaren Bereich dazu, dass die Verbindungsstelle zwischen dem Steuerelement und dem Werkzeug sich relativ zur Drehachse bewegt. Diese Linearbewegung wird durch diese exzentrische Anordnung in eine Drehbewegung des Werkzeugs gegenüber dem zweiten auslenkbaren Bereich umgewandelt. Somit kann auf einfache Weise das Werkzeug mit dem Steuerelement zwischen einer geöffneten und einer geschlossenen Stellung hin- und herbewegt werden.

In einer weiteren Ausgestaltung der Erfindung ist das Steuerelement über ein Kugelgelenk am ersten Ende mit dem ersten Abschnitt bzw. mit dem Befestigungselement oder am zweiten Ende mit dem Werkzeug verbunden.

Eine Kugelpfanne als Paarungsbauteil zu einer Kugel stellt eine produktionstechnisch einfach herzustellende, jedoch sichere gelenkige Verbindung von zwei Bauelementen dar.

Ferner entstehen bei der Verwendung einer Kugelpfanne nur wenige Ecken und Hinterschneidungen, die schwierig zu reinigen sind und auf lange Sicht zu Bakterienansammlungen in dem Instrument führen könnten.

In einer weiteren Ausgestaltung der Erfindung ist das Steuerelement zumindest im Bereich des zweiten Endes als Zahnstange ausgebildet, die über ein Zahngetriebe mit dem Werkzeug in Wirkverbindung steht.

Die Kombination aus einer Zahnstange und einem Zahngetriebe führt durch das Eingreifen von Zahnrädern in die Zahnstange zu einer besonders sicheren Verbindung und somit zu einer sehr effektiven Kraftübertragung.

In einer weiteren Ausgestaltung der Erfindung ist das Instrument als Retraktor ausgebildet.

Die Kombination aus einem auslenkbaren Bereich und einem Werkzeug, das eine offene und eine geschlossene Stellung aufweist, hat sich insbesondere für die Anwendung bei Retraktoren als vorteilhaft erwiesen, da durch den auslenkbaren Bereich zum Beispiel ein Organ von der Rückseite ergriffen und manipuliert werden kann.

Das Werkzeug mit einer offenen und einer geschlossenen Stellung hat den Vorteil, dass zum Beispiel durch Übereinanderlegen verschiedener Retraktorfinger während des Einführens in der geschlossenen Stellung ein kleiner Querschnitt des endoskopischen Instruments geschaffen wird, so dass dieses leicht durch kleine Einschnitte auch in Bereiche des Körpers eines Patienten eingeführt werden kann, die nur schwer zugänglich sind.

In der geöffneten Stellung wiederum bietet das Werkzeug dann eine große Auflagefläche, so dass die beim Manipulieren eines Organs ausgeübte Kraft über einen großen Bereich verteilt wird, wodurch Schäden am Organ durch punktuelle Überbelastung vermieden werden.

In einer weiteren Ausgestaltung weist der Retraktor zumindest zwei Retraktorfinger auf, wobei diese in einer geöffneten Stellung ein Kreuz oder einen Fächer bilden können.

Die Anordnung von zumindest zwei Retraktorfingern, die in geöffneter Stellung ein Kreuz bilden, hat den Vorteil, dass schon mit zwei Retraktorfingern die auf den Retraktor angewendete Kraft über eine große Fläche verteilt werden kann. Somit kann mit nur wenigen Bauteilen eine große effektive Auflagefläche geschaffen werden.

Die Ausführung der Retraktorfinger in der Art, dass sie in geöffneter Stellung ein Kreuz ergeben, hat weiterhin den Vorteil, dass ein solches Kreuz symmetrisch ausgestaltet sein kann, wobei sich der Ansatzpunkt für eine angewendete Kraft in etwa der Mitte des Kreuzes befindet. Somit wird sichergestellt, dass die Kraft gleichmäßig über die gesamten Retraktorfinger verteilt wird.

Die Ausbildung der Retraktorfinger in einer Weise, dass sie in geöffneter Stellung einen Fächer bilden, hat den Vorteil, dass dadurch auf einfache Weise eine durchgängige Auflagefläche geschaffen wird, so dass mögliche Verletzungen durch Eindringen von Gewebe in Zwischenräume zwischen den Retraktorfingern minimiert werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Figur 1: eine perspektivische Ansicht eines erfindungsgemäßen endoskopischen Retraktors,
- Figur 2: eine vergrößerte perspektivische Ansicht des distalen Bereichs des Retraktors von Figur 1 in geschlossener Stellung,
- Figur 3: eine vergrößerte perspektivische Ansicht des distalen Bereichs des Retraktors von Figur 1 in geöffneter Stellung,
- Figur 4: eine vergrößerte Draufsicht auf den distalen Bereich des Retraktors von Figur 1 in geschlossener Stellung,
- Figur 5: einen Teilschnitt durch den distalen Bereich des Retraktors von Figur 1 entlang der Linie V-V von Figur 2,
- Figur 6: eine vergrößerte Frontalansicht des distalen Bereichs des Retraktors von Figur 1 in geöffneter Stellung,
- Figur 7: eine vergrößerte Explosionsdarstellung der Verbindung zwischen dem Werkzeug und dem Steuerelement des Retraktors von Figur 1,
- Figur 8: eine vergrößerte Explosionsdarstellung der Verbindung zwischen dem distalen Bereich und dem Steuerelement des Retraktors von Figur 1,
- Figur 9: eine vergrößerte Seitenansicht des distalen Bereichs des Retraktors von Figur 1 in geschlossener Stellung,
- Figur 10: eine vergrößerte Seitenansicht des distalen Bereichs des Retraktors in Figur 1 in geöffneter Stellung,
- Figur 11: eine vergrößerte Seitenansicht des distalen Bereichs einer weiteren Ausführungsform eines erfindungsgemäßen Retraktors in geschlossener Stellung,
- Figur 12: eine vergrößerte Seitenansicht des distalen Bereichs des Reatraktors von Figur 11 in geöffneter Stellung, und
- Figur 13: eine vergrößerte Draufsicht auf den distalen Bereich des Retraktors von Figur 11 in geöffneter Stellung.

In der vorhergehenden Beschreibung der Zeichnungen und in der nachfolgenden Beschreibung bedeutet der Ausdruck "geschlossene Stellung", dass der zweite auslenkbare Bereich gegenüber dem ersten Bereich nicht ausgelenkt ist, und das Werkzeug sich in geschlossener Stellung befindet. Der Ausdruck "geöffnete Stellung" bedeutet, dass der zweite auslenkbare Bereich gegenüber dem ersten Bereich ausgelenkt ist, und das Werkzeug sich in einer geöffneten Stellung befindet.

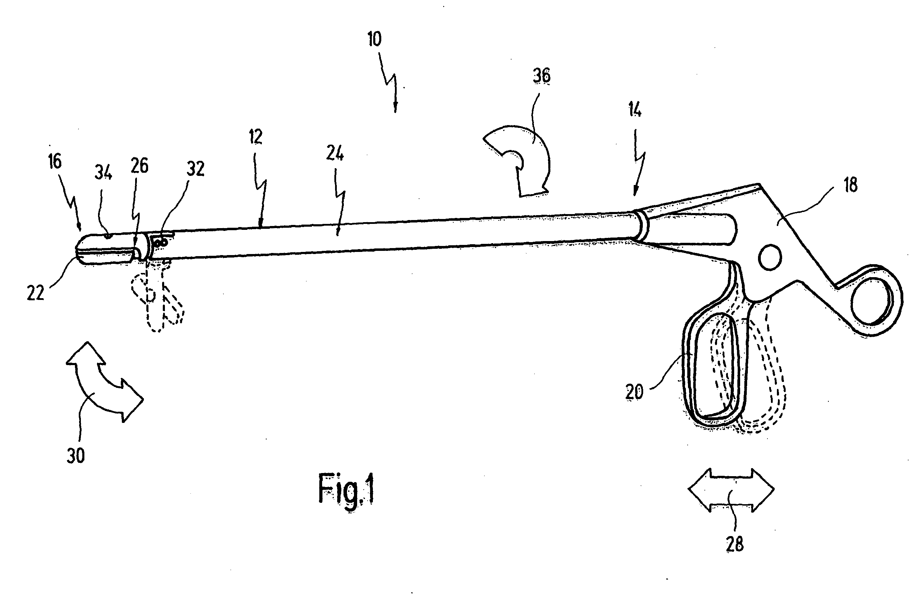

In den Figuren 1 bis 10 ist ein endoskopisches Instrument in seiner Gesamtheit mit der Bezugsziffer 10 bezeichnet. Es handelt sich in diesem Fall um einen endoskopischen Retraktor.

Dieses Instrument 10 weist einen Schaft 12 mit einem proximalen Ende 14 und einem distalen Ende 16 auf. Am proximalen Ende 14 des Schafts 12 ist ein Griff 18 angeordnet, der eine Handhabe 20 aufweist.

Bei dem hier dargestellten Griff 18 handelt es sich um einen scherenartigen Griff, wobei die Handhabe 20 ebenfalls als scherenartiger Griff ausgebildet ist.

Am distalen Ende 16 des Schafts 12 ist ein Werkzeug angeordnet, das hier als Drehkreuz 22 ausgebildet ist.

Der Schaft 12 weist ferner einen ersten Bereich 24 und einen zweiten Bereich 26 auf, der gegenüber dem ersten Bereich 24 auslenkbar ist.

Innerhalb des Schafts 12 verläuft weiterhin ein hier nicht dargestelltes Betätigungselement, das den zweiten auslenkbaren Bereich 26 mit der Handhabe 20 verbindet. Wird nun die Handhabe in Richtung des Doppelpfeils 28 nach rechts in die durch gestrichelte Linien dargestellte Stellung verschoben, wird das Betätigungselement im Schaft 12 nach distal verschoben.

Durch dieses Verschieben wird der zweite auslenkbare Bereich 26 in Richtung des Doppelpfeils 30 um eine Schwenkachse 32, die sich am distalen Ende des ersten Bereichs 24 befindet, um 90° nach unten ausgelenkt. Hierbei wird das Drehkreuz 22 um eine Drehachse 34, die das Drehkreuz 22 mit dem zweiten auslenkbaren Bereich 26 verbindet, um 90° gegenüber dem auslenkbaren Bereich 26 verdreht.

Ein Bewegen der Handhabe in Richtung des Doppelpfeils 28 nach links, also zurück in ihre Ausgangsstellung, bewegt den zweiten auslenkbaren Bereich in Richtung des Doppelpfeils 30 nach oben in seine Ausgangsstellung zurück. Das Drehkreuz 22 wird dabei ebenfalls in seine Ausgangsstellung zurückgedreht.

Um die Flexibilität eines Operateurs zu erhöhen, ist der Schaft 12 weiterhin gegenüber dem Griff 18 um seine Längsachse in Richtung des Pfeils 36 verdrehbar. Durch eine solche Drehung kann die Lage des ausgelenkten zweiten auslenkbaren Bereichs 26 relativ zum Handgriff 18 verändert werden, so dass zum Beispiel ein ausgelenkter zweiter auslenkbarer Bereich nicht wie hier in der Ebene des Griffs 18 ausgelenkt wird, sondern zum Beispiel im rechten Winkel dazu.

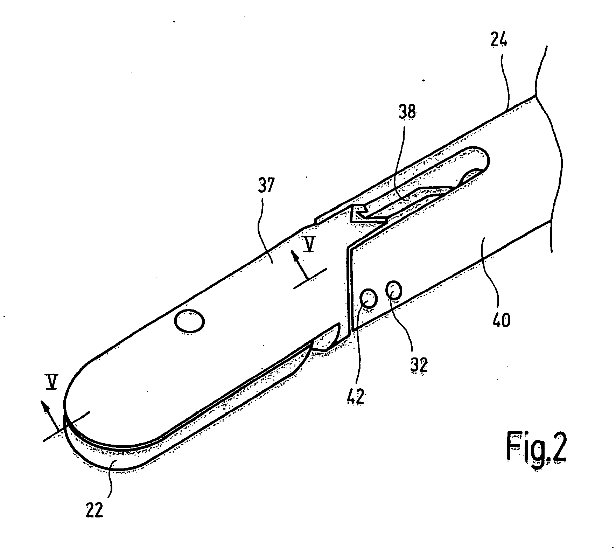

In Figur 2 ist eine perspektivische Ansicht des distalen Abschnitts des endoskopischen Instruments 10 von Figur 1 dargestellt. Es wird hierbei sichtbar, dass der zweite auslenkbare Bereich 26 des Schafts 12 als Schwenkarm 37 ausgebildet ist. Dieser Schwenkarm 37 steht mit dem distalen Ende des Betätigungselements 38 in Wirkverbindung.

Das distale Ende des ersten Bereichs 24 des Schafts 12 ist hierbei in Form einer Gabel 40 ausgebildet, in die der Schwenkarm 37 eingesetzt ist und dort durch einen Stift gehalten wird, der die Schwenkachse 32 bildet.

Ferner weist die Gabel 40 die Hülse 42 auf, die dazu dient, den ersten Bereich 24 des Schafts 12 mit einem Steuerglied zu verbinden.

Das Drehkreuz 22 weist an seinem distalen Ende ein abgerundetes Profil auf, das zusammen mit dem distalen Ende des Schwenkarms 37 das Profil einer Halbkugel ergibt. Dieses halbkugelförmige Profil erleichtert ein atraumatisches Einführen des Instruments 10 in den Körper eines Patienten.

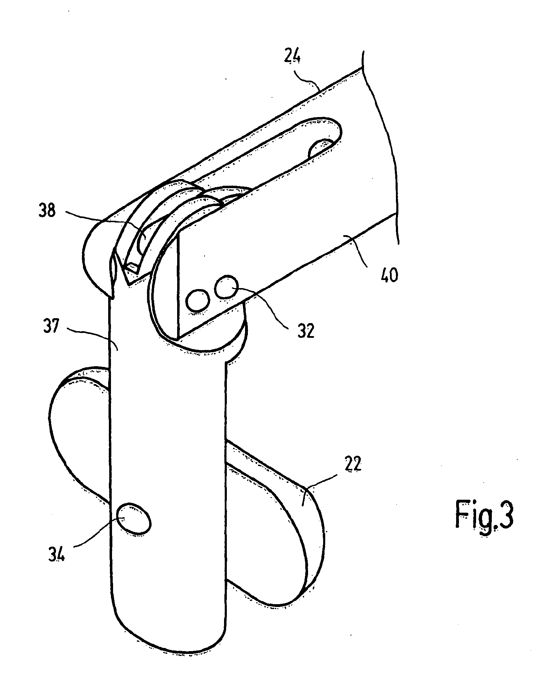

In Figur 3 ist der distale Abschnitt des Instruments 10 in geöffneter Stellung dargestellt. Das Betätigungselement 38 wurde dabei durch Betätigen der Handhabe 20, wie in Figur 1 angedeutet, relativ zum Schaft 12 nach distal verschoben.

Das Betätigungselement 38 ist an einer Stelle mit dem Schwenkarm 37 verbunden, der außerhalb der Schwenkachse 32 liegt. Durch diese exzentrische Anordnung wird die Linearbewegung des Betätigungselements 38 in eine Kreisbewegung des Schwenkarms 34 um die Schwenkachse 32 umgewandelt.

Das Drehkreuz 22 ist in dieser Darstellung gegenüber dem Schwenkarm 37 um 90° um die Drehachse 34 verdreht. Hierbei wird erkennbar, dass das Drehkreuz 22 an seiner Unterseite ein abgerundetes Profil aufweist. Durch dieses Profil werden Verletzungen des umliegenden Gewebes während der Drehbewegung vermieden.

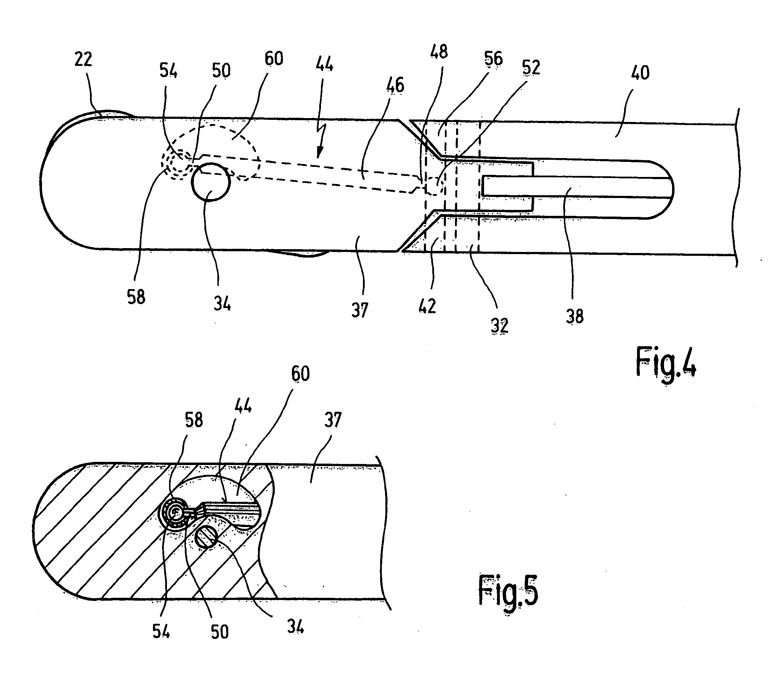

Figur 4 zeigt eine Sicht auf den distalen Abschnitt des Instruments 10, wobei die im Instrument 10 befindlichen Bauteile schematisch durch gestrichelte Linien dargestellt sind.

Der Schwenkarm 37 liegt innerhalb der Gabel 40 und ist mit dieser über die Drehachse 32 drehbar verbunden. Der Schwenkarm 37 ist ferner drehbar mit dem Betätigungselement 38 verbunden. In dem Schwenkarm 37 verläuft ein Steuerelement, das hier als Steuerstange 44 ausgebildet ist. Die Steuerstange 44 ist im Wesentlichen symmetrisch und weist einen stangenförmigen Körper 46 auf, der sich an jeweils gegenüberliegenden Enden zu einem Hals 48, 50 verjüngt, der jeweils mit einem Kopf verbunden ist, der hier durch Kugeln 52, 54 gebildet wird.

Die am proximalen Ende der Stange 44 befindliche Kugel 52 wird ortsfest, aber räumlich kugelartig beweglich eingefasst, indem die Hülse 42 auf einer Seite der Gabel 40 und eine zweite Hülse 56 auf der der Hülse 42 gegenüberliegenden Seite der Gabel 40 kraftschlüssig verbunden werden. Die Kugel 54 am distalen Ende der Steuerstange 44 liegt in einer dritten Hülse 58. Diese dritte Hülse 58 ist in einem unteren Abschnitt drehbar, jedoch kraftschlüssig mit dem Drehkreuz 22 verbunden und verläuft in einem oberen Abschnitt in einer nierenförmigen Ausfräsung 60, die sich etwa halbkreisförmig um die Drehachse 34 im Schwenkarm 37 erstreckt.

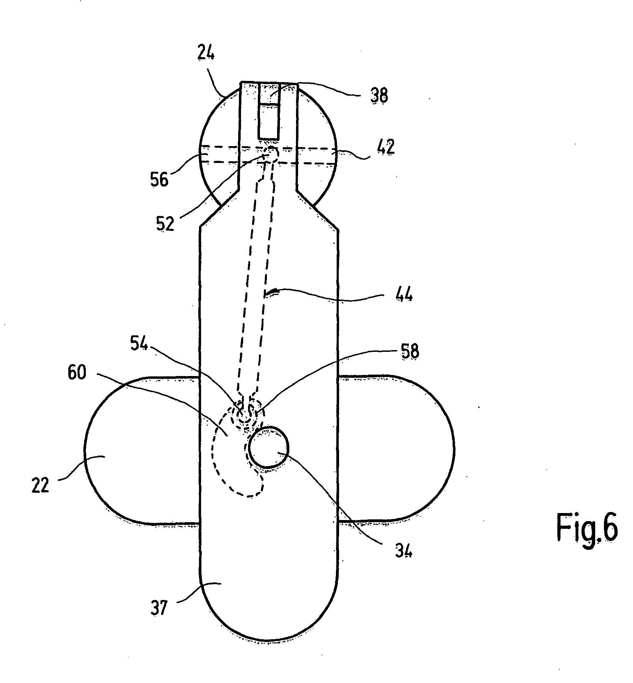

Die dritte Hülse 58 und die Ausfräsung 60 sind in der in Figur 5 dargestellten Schnittzeichnung noch besser zu erkennen. Hierbei wird sichtbar, dass die Kugel 54 der Steuerstange 44 in einer Bohrung der Hülse 58 zu liegen kommt, wobei der Hals 50 der Steuerstange 44 durch einen Schlitz in der Hülse 58 durchgeführt wird.

In Figur 6 ist eine Frontalansicht auf das Instrument von Figur 1 in geöffneter Stellung dargestellt. Um das Instrument in diese Stellung zu überführen, wurde wie in Figur 1 dargestellt die Handhabe 20 gegenüber dem Griff 18 verschoben, wodurch das Betätigungselement 38 relativ zum Schaft nach distal verschoben wurde. Der Schwenkarm 37 ist jetzt gegenüber dem ersten Bereich 24 des Schafts 12 um 90° ausgelenkt.

Der Schwenkarm 34 beschreibt dabei eine Kreisbahn um die hier nicht dargestellte Schwenkachse 32, die in der gleichen Ebene liegt wie die beiden Hülsen 42, 56, jedoch in dieser Darstellung in die Papierebene hinein versetzt angeordnet ist.

Gleichzeitig mit dieser Kreisbewegung des Schwenkarms 37 führt die Steuerstange 44 eine Kreisbewegung um eine Achse aus, die durch die Hülsen 42, 56 definiert wird, die den Kraftschluss zwischen der Kugel 52 und damit der Steuerstange 44 und dem ersten Bereich 24 herstellen.

Nimmt man nun die Drehachse 34 als Referenzpunkt für eine Kreisbewegung des Schwenkarms 37 so wird dadurch eine erste Kreisbahn definiert. Eine zweite Kreisbahn wird durch die Steuerstange 44 definiert, wobei die Kugel 54 eine Kreisbahn beschreibt, deren Mittelpunkt durch die Kugel 52 gebildet wird.

Da die Kreisbahn der Drehachse 34 einen anderen Mittelpunkt aufweist, als die Kreisbahn der Kugel 54, verändert sich während der Bewegung des Schwenkarms 37 der Abstand zwischen der Kugel 54 und der Drehachse 34. Der Abstand zwischen der Drehachse 34 und der Kugel 54 verkürzt sich bis die Kugel 54, gesehen von distal nach proximal in Richtung des Instruments, nach hinten versetzt ist. Die Kugel 54 und die Hülse 58, in der diese kraftschlüssig gehalten wird, bewegen sich dabei in den Ausfräsungen 60 des Schwenkarms 37. Da die Hülse 58 kraftschlüssig mit dem Drehkreuz 22 verbunden ist, wird das Drehkreuz bezogen auf den Schwenkarm 37 entlang der durch die Ausfräsung 60 vorgegebenen Bahn ausgelenkt und gegenüber dem Schwenkarm 37 in einem Winkel von 90° verdreht.

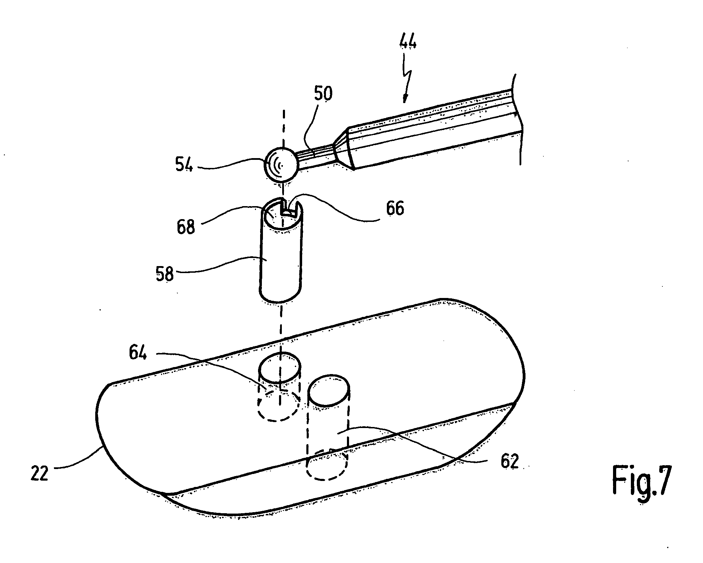

In Figur 7 ist das Drehkreuz 22 näher dargestellt. Das Drehkreuz 22 weist eine zentrale Bohrung 62 auf, die zur Aufnahme der Drehachse dient. Versetzt zur Bohrung 62 ist eine weitere Bohrung 64 in das Drehkreuz 22 eingebracht. Bei dieser Bohrung 64 handelt es sich um eine Sackbohrung.

In diese Sackbohrung wird eine Hülse 58 eingesetzt. Es ist zwar möglich, die Bohrung 64 als durchgängige Bohrung auszuführen, eine Sackbohrung ist aber vorzuziehen, da dadurch die Hülse 58 ohne weitere Haltevorrichtung eingesetzt werden kann, ohne nach unten aus dem Drehkreuz herauszufallen.

In die Hülse 58 wird der distale Abschnitt der Steuerstange 44 eingesetzt, wobei der distale Hals 50 in einem Schlitz 66 der Hülse 58 zu liegen kommt und die Kugel 54 am distalen Ende der Steuerstange 44 in der Bohrung 68 der Hülse 58 zu liegen kommt.

Durch diese Konstruktion ist die Steuerstange 44 kraftschlüssig mit dem Drehkreuz 22 verbunden, ist jedoch gegenüber dem Mittelpunkt der Bohrung 64 noch frei drehbar.

Wird nun das Drehkreuz 22 im Bereich der ersten Bohrung 62 gehalten und auf die kraftschlüssig mit dem Drehkreuz 22 verbundene Steuerstange 44 eine Schub- oder Zugkraft ausgeübt, wird die Hülse 58 und somit die Bohrung 64 auf einer Kreisbahn um die erste Bohrung 62 herumbewegt und somit das Drehkreuz 22 um die Bohrung 62 bzw. eine ggf. darin einzusetzende Drehachse 34 verdreht.

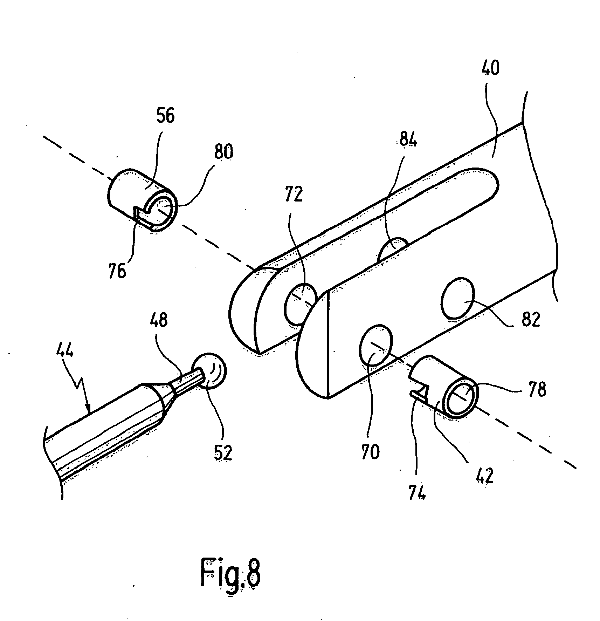

In Figur 8 ist die Gabel 40 dargestellt, die das distale Ende des Schafts 12 bildet. Diese Gabel 40 weist am distalen Ende zwei fluchtende Bohrungen 70, 72 auf. In diese Bohrungen 70, 72 können die Hülsen 42, 56 eingeführt werden. Es wird nun die Steuerstange 44 mit ihrem proximalen Ende in die Gabel 40 eingeführt, so dass die Kugel 52 mit den Bohrungen 70, 72 fluchtet. Danach werden die Hülsen 42, 56 durch die Bohrung 70 bzw. 72 eingeführt. Die Hülsen 42, 56 umfassen dabei die Kugel 52, wobei die Hülse 42 einen Schlitz 74 und die Hülse 56 einen Schlitz 76 aufweisen, die in Flucht miteinander gebracht werden können. Der Hals 48 der Steuerstange 44 kommt in einem durch die Schlitze 74, 76 gebildeten breiten Schlitz zu liegen, und die Kugel 52 kommt in der Bohrung 78 der Hülse 42 bzw. der Bohrung 80 der Hülse 56 zu liegen. Somit ist die Steuerstange 44 mit der Gabel 40 kraftschlüssig, jedoch um eine Achse, die durch die Mittelachse der Bohrung 70 bzw. 72 gebildet wird, verdrehbar verbunden.

Die Gabel 40 weist ferner die miteinander fluchtenden Bohrungen 82, 84 auf, die zum Aufnehmen der Schwenkachse 34 zum Verschwenken des Schwenkarms 37 vorgesehen sind.

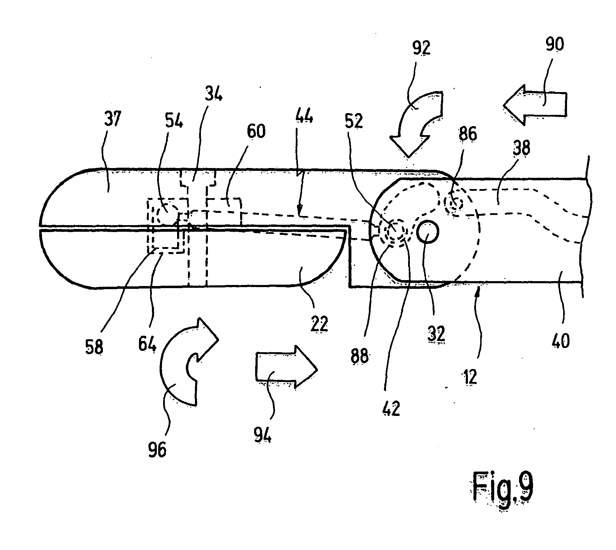

In Figur 9 ist eine Seitenansicht des distalen Endes des Instruments 10 von Figur 1 dargestellt, wobei die innenliegenden mechanischen Bauteile in gestrichelten Linien dargestellt sind. Der Schwenkarm 37 ist hierbei um die Achse 32 verschwenkbar mit der Gabel 40 verbunden. Der Schwenkarm 37 ist ferner durch einen Stift 86 mit dem Betätigungselement 38 beweglich verbunden.

Es wird ferner aus dieser Darstellung deutlich, dass der Schwenkarm 37 eine weitere nierenförmige Ausfräsung 88 aufweist, in der die Hülsen 42, 56 bzw. die am proximalen Ende der Steuerstange 44 angeordnete Kugel 52 aufgenommen sind.

In dieser Zeichnung ist ferner erkennbar, dass die Hülse 58 zum Teil in der Bohrung 64 des Drehkreuzes 22 und zum Teil in der Ausfräsung 60 des Schwenkarms 37 zu liegen kommt. Die Drehachse 34 ist hierbei als Niete ausgebildet.

Wird nun die Handhabe 20 des Instruments 10 betätigt, wird das Betätigungselement 38 relativ zum Schaft 12 in distaler Richtung, also in Richtung des Pfeils 90, verschoben. Hierdurch wird auf den Stift 86, der das Betätigungselement 38 mit dem Schwenkarm 37 verbindet, eine Schubkraft in gleicher Richtung ausgeübt.

Diese Schubkraft führt dazu, dass der Stift 86 auf einer Kreisbahn um die Drehachse 32 herum verschoben wird, wodurch der Schwenkarm 37 eine Schwenkbewegung in Richtung des Pfeils 92 ausführt. Durch diese Schwenkbewegung bewegt sich die Drehachse 34 auf einer Kreisbahn um die Schwenkachse 32. Ferner bewegt sich der Schwenkarm 37 über die nierenförmige Ausfräsung 88 um die Hülsen 42, 56 bzw. die Kugel 52 der Steuerstange 44.

Die Position der Kugel 52 relativ zur Gabel 40 bzw. zur Schwenkachse 32 bleibt dabei unverändert. Da die Schwenkachse 34 mit dem Drehkreuz 22 in Wirkverbindung steht und dieses Drehkreuz 22 mit der Kugel 54 in Wirkverbindung steht, wird die Kugel 54 auf einer Kreisbahn um die Kugel 52 herumgeführt. Es handelt sich hierbei also um zwei exzentrische Kreisbewegungen.

Bei solchen exzentrischen Kreisbewegungen verändert sich die relative Lage der Kreisbahnen zueinander, also in diesem Fall verändert sich die relative Lage der Kugel 54 der Steuerstange 44 gegenüber der Drehachse 34 des Schwenkarms 37. Die Kugel 54 der Steuerstange 44 wird hierbei relativ zur Drehachse 34 des Schwenkarms 37 in Richtung des Pfeils 94 zur Achse 32 hin versetzt.

Durch diese Relativbewegung der Kugel 54 wird über die Hülse 58 und die Bohrung 64 auf das Drehkreuz 22 eine Zugkraft ausgeübt. Diese Zugkraft bewegt die Kugel 54 auf einer Kreisbahn um die Drehachse 34, wodurch das Drehkreuz 22, das kraftschlüssig mit der Kugel 54 verbunden ist, um die Drehachse 34 eine Drehbewegung in Richtung des Pfeils 96 ausführt.

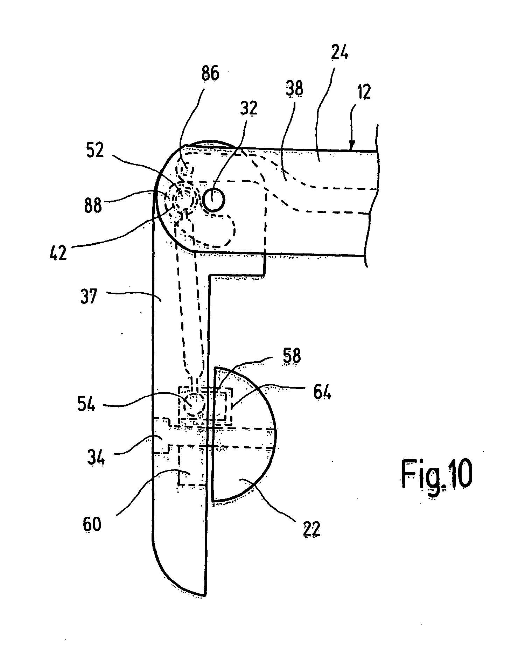

Figur 10 zeigt die gleiche Ansicht wie Figur 9, allerdings in geöffneter Stellung des endoskopischen Instruments 10. Es wird hierbei sichtbar, dass die Kugel 54 gegenüber der Drehachse 34 in der Ausfräsung 60 versetzt ist. Die somit auf die Hülse 58 und damit die Bohrung 64 ausgeübte Zugkraft hat das Drehkreuz 22 gegenüber dem Schwenkarm 37 um 90° um die Drehachse 34 verdreht.

Es ist weiterhin sichtbar, dass sich die Ausfräsung 88 um die Kugel 52 bzw. die Hülse 42 herumbewegt hat.

In dieser geöffneten Stellung ist sowohl der Schwenkarm angewinkelt als auch das Drehkreuz gegenüber dem Schwenkarm ausgelenkt.

Der Retraktor kann in dieser Stellung, zum Beispiel bei einer minimalinvasiven Operation, dazu verwendet werden, ein Organ, zum Beispiel die Leber, von hinten zu ergreifen und diese zu bewegen, um zum Beispiel eine Untersuchung der Rückseite der Leber zu ermöglichen.

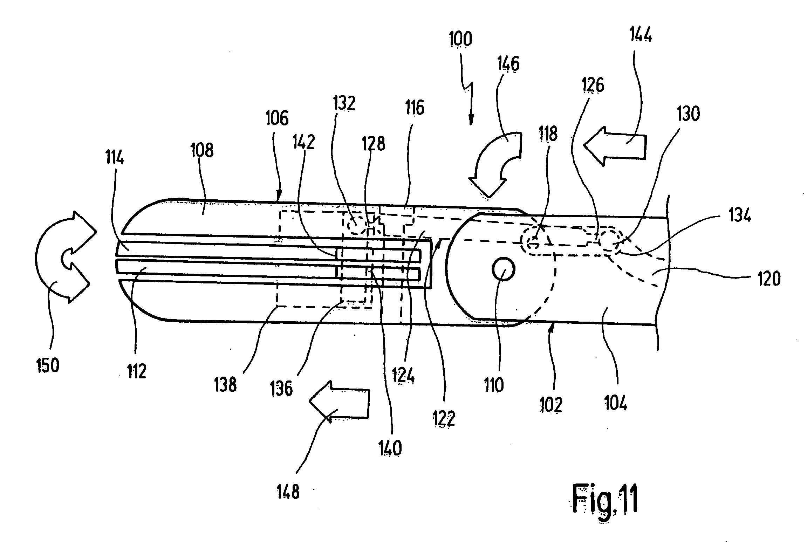

In Figur 11 ist eine Seitenansicht eines distalen Endes einer weiteren Ausführungsform eines endoskopischen Retraktors dargestellt, wobei die innenliegenden mechanischen Bauteile in gestrichelten Linien dargestellt sind.

Der endoskopische Retraktor weist hierbei einen Schaft 100 auf, der einen ersten Bereich 102, der hier als Gabel 104 ausgebildet ist, und einen zweiten auslenkbaren Bereich 106 aufweist, der hier als Schwenkarm 108 ausgebildet ist.

Der Schwenkarm 108 liegt im distalen Ende der Gabel 104 und ist mit dieser über eine Schwenkachse 110 verschwenkbar verbunden, die hier in Form eines Stifts ausgebildet ist.

Ein am zweiten auslenkbaren Bereich 106 des Schafts 100 angeordnetes Werkzeug des endoskopischen Retraktors ist hier in Form eines Fächers ausgebildet, der Retraktorfinger 112 und 114 aufweist. Die Retraktorfinger 112, 114 sind über eine Schwenkachse 116, die hier als Schraube ausgebildet ist, drehbar in dem Schwenkarm 108 gelagert. Das distale Ende des Schwenkarms 108 und die distalen Enden der Retraktorfinger 112 und 114 sind hierbei abgerundet und bilden ein etwa halbkugelförmiges Profil, welches ein möglichst atraumatisches Einführen des Instruments in den Körper ermöglicht.

An seinem proximalen Ende ist der Schwenkarm 108 außerdem durch einen Stift 118 mit einem Betätigungselement 120 verbunden. Das Betätigungselement 120 ist an seinem proximalen Ende auf eine Weise analog zu dem Instrument 10 aus Figur 1 mit einer Handhabe verbunden und kann durch diese axial bewegt werden.

Durch eine axiale Bewegung des Betätigungselements 120 wird eine Auslenkung des Schwenkarms 108 gegenüber der Gabel 104 hervorgerufen. Hierzu befindet sich der Stift 118 in einer exzentrischen Position zur Schwenkachse 110. Wird nun das Betätigungselement 120 in axialer Richtung bewegt, führt der Stift 118 eine Kreisbewegung um die Schwenkachse 110 aus, wodurch der Schwenkarm 108 gegenüber der Gabel 104 ausgelenkt wird.

Das Betätigungselement 120 ist ferner mit einem Steuerelement verbunden, das hier als Steuerstange 122 ausgebildet ist. Die Steuerstange 122 ist im Wesentlichen symmetrisch und weist einen Stangenkörper 124 auf, der sich jeweils in Richtung seines proximalen bzw. distalen Endes zu einem Stangenhals 126 bzw. 128 verjüngt.

Den Stangenhälsen 126, 128 schließen sich proximal bzw. distal Köpfe der Steuerstange 122 an, die hier als Kugeln 130 bzw. 132 ausgebildet sind.

Die sich am proximalen Ende der Steuerstange 122 befindliche Kugel 130 liegt in einer geschlitzten Hülse 134, die fest, jedoch drehbar mit dem Betätigungselement 120 verbunden ist, wobei der proximale Hals 126 der Steuerstange 122 in einem Schlitz der Hülse 134 zu liegen kommt.

Die Kugel 132 am distalen Ende der Steuerstange 122 kommt in einer geschlitzten Hülse 136 zu liegen. Diese geschlitzte Hülse 136 ist verschiebbar in einer Ausfräsung 138 im Schwenkarm 108 angeordnet. Die geschlitzte Hülse 136 ist ferner verschiebbar in, bezogen auf die Ausfräsung 138 des Schwenkarms 108, schräg angeordneten Ausfräsungen 140 bzw. 142 in den Retraktorfingern 112 bzw. 114 angeordnet. Die Kugel 132 am distalen Ende der Steuerstange 122 kommt dabei in der Hülse 136 zu liegen, während der Stangenhals 128 am distalen Ende der Steuerstange 122 in einem Schlitz der Hülse 136 zu liegen kommt.

Wird nun mit Hilfe der hier nicht dargestellten Handhabe das Betätigungselement 120 in Richtung des Pfeils 144 in Richtung des Schafts 100 von proximal nach distal bewegt, beschreibt der Stift 118 eine Kreisbahn um die Schwenkachse 110 in Richtung des Pfeils 146. Hierdurch kommt es zu einer Auslenkung des Schwenkarms 108 gegenüber der Gabel 104, ebenfalls in Richtung des Pfeils 146.

Dadurch, dass die Steuerstange 122 über die Hülse 134 und die Kugel 130 fest mit dem Betätigungselement 120 verbunden ist, führt die Steuerstange 122, bezogen auf den Schwenkarm 108, ebenfalls eine Linearbewegung in Richtung des Pfeils 148 aus. Hierdurch wird die über die Kugel 132 fest mit der Steuerstange 122 verbundene Hülse 136 in der Ausfräsung 138 in distaler Richtung, also ebenfalls in Richtung des Pfeils 148, verschoben. Hierbei bewegt sich die Hülse 136 in den, bezogen auf die Ausfräsung 180, schräg angeordneten Ausfräsungen 140 und 142 in den Retraktorfingern 112 bzw. 114. Durch diese Bewegung werden die Retraktorfinger 112, 114 um die Drehachse 116, bezogen auf den Schwenkarm 108, in Richtung des Doppelpfeils 150, in jeweils entgegengesetzte Richtung verdreht.

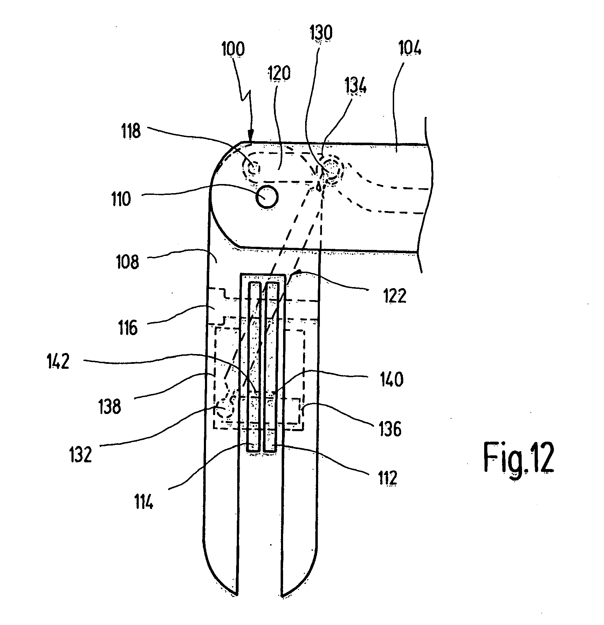

In Figur 12 ist der Retraktor von Figur 11 in geöffneter Stellung dargestellt. Das Betätigungselement 120 wurde zum Beispiel gegenüber der Schwenkachse 110 in Richtung des distalen Endes des Schafts 100 verschoben. Der Stift 118 hat dabei eine Kreisbewegung um die Schwenkachse 110 ausgeführt, wodurch der Schwenkarm 108 in einen 90° Winkel gegenüber der Gabel 104 ausgelenkt wurde.

Die Hülse 134 und dadurch die Kugel 130 am proximalen Ende der Steuerstange 122 wurden ebenfalls gegenüber der Schwenkachse 110 in distaler Richtung verschoben. Ferner hat die Kugel 132 am distalen Ende der Steuerstange 122, da diese fest mit dem Schwenkarm 108 verbunden ist, eine Kreisbewegung um die Kugel 130 am proximalen Ende der Steuerstange 122 bzw. um die Hülse 134 ausgeführt.

Diese kombinierte Linear- und Kreisbewegung führt dazu, dass die Kugel 132 am distalen Ende der Steuerstange 122, bezogen auf die Drehachse 116, nach distal verschoben wurde. Dadurch wurde die Hülse 136 in der Ausfräsung 138 des Schwenkarms 108 verschoben. Während dieser Linearbewegung der Hülse 136 in der Ausfräsung 138 gleitet die Hülse 136 durch die schrägen Ausfräsungen 140 und 142 der Retraktorfinger 112 bzw. 114, die jeweils in unterschiedliche Richtungen Schrägen aufweisen, wodurch diese um die Drehachse 116 gegenüber dem Schwenkarm 108 jeweils in unterschiedliche Richtungen verdreht wurden.

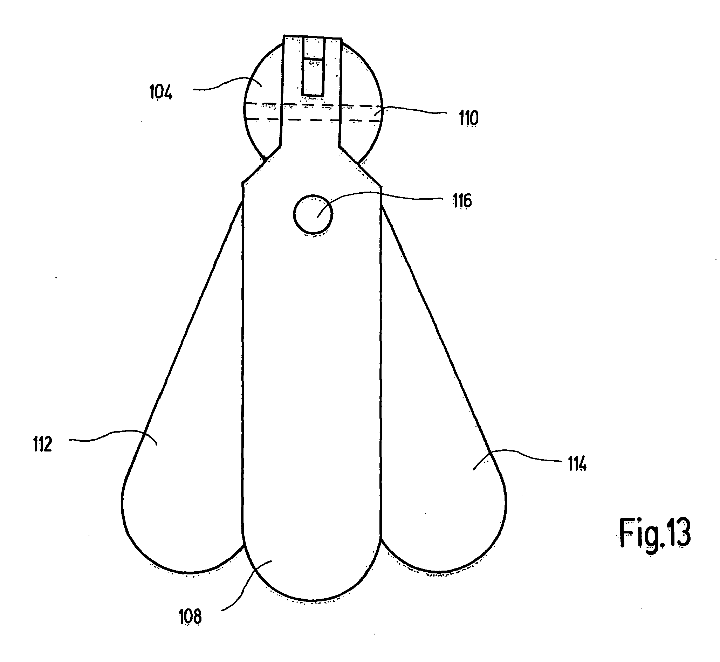

Figur 13 stellt diese geöffnete Stellung noch einmal in der Draufsicht dar. Der Schwenkarm 108 wurde gegenüber der Gabel 104 um die Schwenkachse 110 in etwa einem 90° Winkel ausgelenkt. Die Retraktorfinger 112 und 114 wurden jeweils in unterschiedliche Richtungen gegenüber den Schwenkarm 108 um die Drehachse 116 verdreht. Hierdurch nehmen der Schwenkarm 108 und die beiden Retraktorfinger 112 und 114 eine fächerförmige Konfiguration an.

Durch diese Konfiguration wird die Auflagefläche des Schwenkarms 108 deutlich erhöht und somit eine durch den Schwenkarm 108 zum Beispiel auf ein Organ ausgeübte Kraft über einen deutlich größeren Bereich verteilt, wodurch Verletzungen des Organs durch eine lokale Überlastung vermieden werden.

## Patentansprüche

1. Endoskopisches Instrument mit einem Schaft (12; 100), der ein proximales und ein distales Ende (14, 16) aufweist,
wobei am proximalen Ende (14) des Schafts (12; 100) ein Griff (18) mit einer Handhabe (20) und am distalen Ende (16) des Schafts (12; 100) ein Werkzeug angeordnet ist, das zumindest eine geöffnete und eine geschlossene Stellung aufweist,
wobei der Schaft (12; 100) zumindest zwei Bereiche aufweist, einen ersten Bereich (24; 102) und einen zweiten gegenüber dem ersten auslenkbaren Bereich (26; 106),
wobei der zweite auslenkbare Bereich (26; 106) das Werkzeug aufweist, und
wobei das Instrument (10) ferner ein Betätigungselement (38; 120) aufweist, über das die Handhabe (20) mit dem zweiten auslenkbaren Bereich (26; 106) in Wirkverbindung steht und mit dem eine Auslenkung des zweiten auslenkbaren Bereichs (26; 106) auslösbar ist,
**dadurch gekennzeichnet, dass** der erste Bereich (24; 104) oder das Betätigungselement,(38; 120) und das Werkzeug über ein Steuerelement (44, 122) verbunden sind, mittels dem das Werkzeug im Laufe einer Auslenkung des zweiten auslenkbaren Bereichs (26; 106) zwischen der geschlossenen und der offenen Stellung hin- und herbewegbar ist,
so dass durch die Bewegung des Betätigungselements (38; 120) sowohl die Auslenkung des zweiten auslenkbaren Bereichs (26; 106) als auch ein Öffnen/Schließen des Werkzeugs bewerkstelligbar ist.

2. Instrument nach Anspruch 1, **dadurch gekennzeichnet, dass** das Steuerelement als Steuerstange (44; 122) ausgebildet ist.

3. Instrument nach Anspruch 1 oder 2, **dadurch gekennzeichnet, dass** der erste Bereich (24; 104) und der zweite auslenkbare Bereich (26; 106) über eine Schwenkachse (32; 110) verbunden sind.

4. Instrument nach einem der Ansprüche 1 bis 3, **dadurch gekennzeichnet, dass** das Werkzeug um eine Drehachse (34; 116) bewegbar am zweiten auslenkbaren Bereich (26; 106) angeordnet ist.

5. Instrument nach Anspruch 3 oder 4, **dadurch gekennzeichnet, dass** das ein erstes Ende des Steuerelements an einer Stelle mit dem ersten Bereich (24; 104) verbunden ist, die im Abstand zur Schwenkachse (32; 110) liegt.

6. Instrument nach einem der Ansprüche 1 bis 5, **dadurch gekennzeichnet, dass** das ein zweites Ende des Steuerelements an einer Stelle mit dem Werkzeug verbunden ist, die im Abstand zur Drehachse (34; 106) liegt.

7. Instrument nach einem der Ansprüche 1 bis 6, **dadurch gekennzeichnet, dass** das Steuerelement am ersten Ende über eine Kugelpfanne mit dem ersten Bereich (24; 104) verbunden ist.

8. Instrument nach einem der Ansprüche 1 bis 7, **dadurch gekennzeichnet, dass** das Steuerelement am zweiten Ende über eine Kugelpfanne mit dem Werkzeug verbunden ist.

9. Instrument nach einem der Ansprüche 1 bis 7, **dadurch gekennzeichnet, dass** das Steuerelement zumindest im Bereich des zweiten Endes als Zahnstange ausgebildet ist, die über ein Zahngetriebe mit dem Werkzeug in Wirkverbindung steht.

10. Instrument nach einem der Ansprüche 1 bis 9, **dadurch gekennzeichnet, dass** das Instrument (10) als Retraktor ausgebildet ist.

11. Instrument nach Anspruch 10, **dadurch gekennzeichnet, dass** der Retraktor zumindest zwei Retraktorfinger (112, 114) aufweist.

12. Instrument nach Anspruch 11, **dadurch gekennzeichnet, dass** die Retraktorfinger in einer geöffneten Stellung ein Kreuz bilden.

13. Instrument nach Anspruch 11, **dadurch gekennzeichnet, dass** die Retraktorfinger (112, 114) in einer geöffneten Stellung einen Fächer bilden.

## Claims

1. Endoscopic instrument having a shaft (12; 100), comprising a proximal end (14) and a distal end (16),
a grip (18) having a handle (20) being arranged at the proximal end (14) of the shaft (12; 100) and a tool being arranged at the distal end (16) of the shaft (12; 100) which has at least an open position and a closed position,
the shaft (12; 100) comprising at least two sections, a first section (24; 102) and a second section (26; 106), which can be articulated with respect to the first section,
the second, articulating section (26; 106) comprising the tool, and
the instrument (10) further comprising an actuating element (38; 120), by means of which the handle (20) is in operative connection with the second, articulating section (26; 106) and by means of which an articulation of the second, articulating section (26; 106) can be initiated,
**characterized in that** the first section (24; 104) or the actuating element (38, 120) and the tool are connected by means of a control element (44, 122), by means of which the tool can be moved back and forth between the closed position and the open position in the course of an articulation of the second, articulating section (26; 106),
so that both the articulation of the second, articulating section (26; 106) and an opening/closing of the tool can be accomplished by the movement of the actuating element (38; 120).

2. Instrument according to Claim 1, **characterized in that** the control element is designed as a control rod (44; 122).

3. Instrument according to Claim 1 or 2, **characterized in that** the first section (24; 104) and the second, articulating section (26; 106) are connected by means of a swivel axis (32; 110).

4. Instrument according to one of Claims 1 to 3, **characterized in that** the tool is arranged movably about a pivot axis (34; 116) on the second, articulating section (26; 106).

5. Instrument according to Claim 3 or 4, **characterized in that** a first end of the control element is connected to the first section (24; 104) at a location which lies at a distance from the swivel axis (32; 110).

6. Instrument according to one of Claims 1 to 5, **characterized in that** a second end of the control element is connected to the tool at a location which lies at a distance from the pivot axis (34; 106).

7. Instrument according to one of Claims 1 to 6, **characterized in that** the control element is connected at the first end to the first section (24; 104) by means of a ball socket.

8. Instrument according to one of Claims 1 to 7, **characterized in that** the control element is connected at the second end to the tool by means of a ball socket.

9. Instrument according to one of Claims 1 to 7, **characterized in that** the control element is designed at least in the region of the second end as a toothed rack which is in operative connection with the tool by means of a gear mechanism.

10. Instrument according to one of Claims 1 to 9, **characterized in that** the instrument (10) is designed as a retractor.

11. Instrument according to Claim 10, **characterized in that** the retractor comprises at least two retractor fingers (112, 114).

12. Instrument according to Claim 11, **characterized in that**, in an open position, the retractor fingers form a cross.

13. Instrument according to Claim 11, **characterized in that**, in an open position, the retractor fingers (112, 114) form a fan.

## Revendications

1. Instrument endoscopique avec une tige (12 ; 100), qui présente une extrémité proximale et distale (14, 16),
une poignée (18) avec une manette (20) étant disposée sur l'extrémité proximale (14) de la tige (12 ; 100) et un outil présentant au moins une position ouverte et une position fermée étant disposé sur l'extrémité distale (16) de la tige (12; 100),
la tige (12 : 200) présentant au moins deux zones, une première zone (24 ; 102) et une deuxième zone susceptible d'être déviée (26 ; 106) par rapport à la première,
la deuxième zone susceptible d'être déviée (26 : 106) présentant l'outil, et
l'instrument (10) présentant par ailleurs un élément de manoeuvre (38; 120) par l'intermédiaire duquel la manette (20) est en interaction avec la deuxième zone susceptible d'être déviée (26 ; 106) et à l'aide duquel une déviation de la deuxième zone susceptible d'être déviée (26 ; 106) peut être déclenchée,
**caractérisé en ce que** la première zone (24 ; 104) ou l'élément de manoeuvre (38 ; 120) et l'outil sont reliés par l'intermédiaire d'un élément de commande (44, 122), au moyen duquel, au cours d'une déviation de la deuxième zone susceptible d'être déviée (26 ; 106), l'outil est susceptible d'être déplacé de part et d'autre entre la position fermée et la position ouverte,
pour que suite au déplacement de l'élément de manoeuvre (38 ; 120), on puisse procéder aussi bien à la déviation de la deuxième zone susceptible d'être déviée (26 ; 106) qu'à l'ouverture/fermeture de l'outil.

2. Instrument selon la revendication 1, **caractérisé en ce que** l'élément de commande est conçu sous la forme d'une barre de commande (44 ; 122).

3. instrument selon la revendication 1 ou 2, **caractérisé en ce que** la première zone (24 ; 104) et la deuxième zone susceptible d'être déviée (26 ; 106) sont reliées par l'intermédiaire d'un axe de pivotement (32; 110).

4. Instrument selon l'une quelconque des revendications 1 à 3, **caractérisé en ce que** l'outil est disposé sur la deuxième zone susceptible d'être déviée (26 ; 106), de façon déplaçable autour d'un axe de rotation (34 ; 116).

5. Instrument selon la revendication 3 ou 4, **caractérisé en ce que** la première extrémité de l'élément de commande est reliée avec la première zone (24 ; 104) en un point qui se situe à distance de l'axe de pivotement (32 ; 110).

6. Instrument selon l'une quelconque des revendications 1 à 5, **caractérisé en ce que** la deuxième extrémité de l'élément de commande est relié avec l'outil en un point qui se situe à distance de l'axe de rotation (34 ; 106).

7. Instrument selon l'une quelconque des revendications 1 à 6, **caractérisé en ce que** l'élément de commande est relié sur la première extrémité avec la première zone (24 ; 104), par l'intermédiaire d'un coussinet sphérique.

8. Instrument selon l'une quelconque des revendications 1 à 7, **caractérisé en ce que** l'élément de commande est relié sur la deuxième extrémité avec l'outil, par l'intermédiaire d'un coussinet sphérique.

9. Instrument selon l'une quelconque des revendications 1 à 7, **caractérisé en ce que**, au moins dans la zone de la deuxième extrémité, l'élément de commande est conçu sous la forme d'une crémaillère, qui par l'intermédiaire d'une transmission par engrenage est avec en interaction avec l'outil.

10. Instrument selon l'une quelconque des revendications 1 à 9. **caractérisé en ce que** l'instrument (10) est conçu sous la forme d'un rétracteur.

11. Instrument selon la revendication 10, **caractérisé en ce que** le rétracteur présente au moins deux doigts de rétracteur (112, 114).

12. Instrument selon la revendication 11, **caractérisé en ce que**, dans une position ouverte, les doigts de rétracteur forment une croix.

13. Instrument selon la revendication 11, **caractérisé en ce que** dans une position ouverte, les doigts de rétracteur (112, 114) forment un éventail.
